# EUROPEAN PATENT SPECIFICATION

(11) **EP 1 746 980 B1**
(45) Date of publication and mention of the grant of the patent: **02.11.2011**
(21) Application number: 05743110.8
(22) Date of filing: 04.05.2005
(51) Int. Cl.: A61K 9/62, A61K 9/26

(54) **PHARMACEUTICAL DOSAGE FORM COMPRISING PELLETS AS WELL AS ITS MANUFACTURING PROCESS**
NEUE PHARMAZEUTISCHE DOSIERFORM UND HERSTELLUNGSVERFAHREN
FORME PHARMACEUTIQUE COMPRENANT DES PELLETS ET PROCÉDÉ DE FABRICATION

(30) Priority: 07.05.2004 EP 04101982
(43) Date of publication of application: 31.01.2007
(73) Proprietor: Nycomed GmbH, 78467 Konstanz (DE)
(72) Inventor: ANSTETT-KLEIN, Isabel, 70771 Leinfelden - Echterdingen (DE); SCHILLER, Marc, 78315 Radolfzell (DE)
(74) Representative: Kratzer, Bernd
(86) International application number: PCT/EP2005/052066
(87) International publication number: WO 2005/107721

(56) References cited:
- EP-A- 1 108 425
- WO-A-97/02020
- WO-A-99/48498
- WO-A-2004/013126
- US-A- 5 385 739

## Description

### Technical field

The present invention relates to the field of pharmaceutical technology and describes a manufacturing process for pellets containing an active ingredient by spraying a suspension of a disintegrant containing the active ingredient on starter pellets. The invention also relates to the pellets and dosage forms obtained by such a process.

### Prior art

It is generally known in pharmaceutical technology to use starch and particularly pregelatinized starch as disintegrant for granulation of a mixture of active ingredients and several excipients in suspension or using it as a dry substance for this intention. It is also generally known to use starch for manufacturing nonpareille particles in dry status. WO 98/52564 for examples discloses the coating of nonpareille seeds in a centrifugal coater with a dusting powder composed of active drug, sucrose, com starch and talcum, while spraying a hydroxypropyl methyl cellulose Solution.

It is also generally known to produce oral dosage forms of active ingredients using a suspension of starch as granulation liquid.

EP 1108425 is related to multi-unitary pharmaceutical preparations containing substituted benimidazoles. The preparations are pellet preparations with an inert core which is coated with an active layer containing the benzimidazole and excipients, mixed in suitable proportions in order to allow the disaggregation of the formulations and dissolution of the active ingredient. The active layer in tum is coated with an insulating layer of a strictly polymeric nature, this layer being coted in tum with an enteric layer.

EP 773025 is related to an oral pharmaceutical preparation containing an acid-labile benzimidazole compound which comprises a nucleous formed by coating a spherical inert core with the benzimidazole, hydroxypropylmethylcellulose and talc, an inert coating disposed on said nucleous, formed by hydroxypropylmethylcellulose, titanium dioxide and talc, an outer layer disposed on the previous coating comprising an enteric coating containing co-polymerized methacrylic acid/methacrylic acid methyl ester, triethylcitrate and talc.

US 6123962 is related to a granule which comprises nonpareils coated with a mixture of a benzimidazole compound, a basic inorganic salt stablizing agent and an additive.

US 6346269 is related to a method for preparing an oral formulation containing acid-sensitive drugs, including at least the following step: spreading a solution or a suspension containing at least stabilizers, solvents and acid-sensitive drugs or its pharmaceutically acceptable salts onto a core made from one or more excipients, and then drying the core to make an active ingredient layer over the core. Also disclosed is the oral formulation made by this method.

US 5385739 is related to a stable formulation of omeprazole microgranules containg a neutral core consisting of sugar and starch, characterized in that it contains an active layer consisting of a dilution of omeprazole in mannitol in substantially equal amounts.

WO 99/48498 is related to an oral pharmaceutical formulation comprising granules having an inert core coated with a layer, comprising an benzimidazole having anti-ulcer activity, a disintegrant and a surfactant in a matrix of a melt coating substance essentially consisting of one or more esters of glycerol and fatty acids, a separating layer and an enteric coating layer, and a process for the preparation of such formulation using a melt coating technique for the preparation of the benzimidazole containing layer.

WO 9702020 is related to an oral pharmaceutical composition of pantoprazole in pellet or tablet form, wherein the pantoprazole is at least partly in slow release form.

WO 2004/098594 is related to dosage forms for oral administration of the magnesium salt of pantoprazole.

WO 2004/098577 is related to dosage forms for oral administration of the magnesium salt of (S)-pantoprazole.

### Description of the invention

Surprisingly it has been found now, that by spraying a suspension of a disintegrant containing the active ingredient on starter pellets, active ingredient layered pellet cores can be obtained which have an improved release profile of the active ingredient. These pellet cores show a particular faster and increased release of the active ingredient as compared to conventionally prepared pellet cores.

The invention therefore relates to pellets comprising starter pellets layered with a layer comprising an active ingredient, a disintegrant and optionally other pharmaceutically acceptable excipients. According to the invention, the layer is formed by spraying a suspension of a disintegrant containing the active ingredient and optionally other pharmaceutically acceptable excipients on starter pellets, whereby the disintegrant is pregelatinized com or maize starch and said active ingredient is a pantoprazole magnesium salt

Starter pellets (also referred to as seed pellets or nonpareil seeds herein), which can be used as starter partides in the spraying process according to the invention, are based on materials known to the person skilled in the art in pharmaceutical technology such as cellulose, sucrose, starch, hydroxypropyl methyl cellulose (HPMC), whereby sucrose and starch are preferred materials. The particle size of the seed pellets is preferably in the range of 0.25 and 1.4 mm, preferably between 0.4 and 1.3 mm and most particularly preferred in the range of 0.6 and 1.0 mm.

The disintegrant according to the invention can be pregelatinized com or maize starch (e.g. Starch 1500® or Star-X®), whereby said pregelatinized starch is employed as disintegration aid. The term pregelatinized starch in connection with the invention also includes partly pregelatinized starch.

The quantity (in percent of weight based on the active pellet core without further optional coatings) of pregelatinized corn or maize starch is preferably in the range of 0.5 and 5%, particularly preferred In the range of 1.0 and 4.0% and most preferred between 2.0 and 3.5%. Active pellet core in this connection refers to a pellet in connection with the invention consisting of a starter pellet layered with a layer of an active ingredient, pregelatinized com or maize starch and optionally other pharmaceutically acceptable excipients.

Preferably an aqueous suspending agent is used. The concentration of pregelatinized corn or maize starch in the suspension containing the active ingredient used for spraying on the starter pellets is preferably in the range of 0.5 and 5% and particularly preferred in the range of 1.0 and 3.0% in percent of weight based on the spraying suspension.

The average particle size of the used pregelatinized com or maize starch or partially pregelatinized com or maize starch (determined by suitable methods) is preferably in the range of 10 and 200 µm, particularly preferred in the range of 40 and 120 µm and most preferred between 60 and 100 µm.

The proportion (in percent by weight based on the finished dosage form) of pregelatinized corn or maize starch as disintegration aid is preferably in the range of 0.5 and 5.0% and most preferred between 1.0 and 3.0%.

The pellet core may contain additional excipients such as binders, stabilizers, additional disintegrants, surfactant and wetting agents

Suitable binders which can be used for layering the suspension of the disintegrant containing the active ingredient onto the starter pellet are polyvinylpyrrolidone (PVP), hydroxypropylmethylcellulose, hydroxypropylcellulose, sodium carboxymethylcellulose, gelatine, whereby PVP is preferred. The polyvinylpyrrolidone (PVP) employed as binder according to the invention can be of molecular weight in the range of 2.000 - 1.500.000. In one embodiment PVP 90 (average molecular weight about 1.000.000 -1 _500.000) or PVP in the range of from 600 000 to 700 000 can be mentioned as preferred. In another embodiment of the invention the PVP is a water-soluble PVP with a low average molecular weight and is preferably used as binder in the dosage form. Low average molecular weight in connection with the invention refers to PVP with an average molecular weight below 300 000, preferably below 100 000, particularly preferably below 70 000, more particularly preferably below 60 000, most particularly preferred below 40 000. Examples, which may be mentioned, are Kollidon 12 PF (molecular weight 2 000-3 000), Kollidon 17 PF (molecular weight 7 000-11 000). Kollidon 25 (molecular weight 28 000-34 000) and Kollidon 30 (molecular weight 44 000-54 000), whereby Kollidon 25 is preferred.

Wetting agents or surfactants, which can be used in the pellet core, preferably refer to synthetic tensides (such as polysorbate, spans, brij), sulfate- and sulfonate salts of fatty acids (such as sodium dodecylsulfate), non-ionic tensides (such as poloxamer) and glycerol esters of fatty acids. In a preferred embodiment SDS (sodium dodecylsulfate) is present.

Besides binder, other ancillary substances, in particular lubricants and anti-sticking agents, and other disintegration aids, are used in the manufacture of the nonpareille pellet cores. Examples of lubricants and anti-sticking agents, which may be mentioned, are higher fatty acids and their alkali metal and alkaline-earth-metal salts, such as calcium stearate. Another suitable lubricant is talc. Other suitable disintegration aids, in particular, chemically inert agents, which may be mentioned as preferred, are crosslinked polyvinylpyrrolidone, crosslinked sodium carboxymethylcelluloses and sodium starch glycolate.

In another embodiment the invention relates to a process for manufacturing the pellets according to the invention comprising spraying a suspension of pregelatinized corn or maize starch containing the active ingredient and optionally other excipients on starter pellets. Preferably the thus obtained layered pellets are subsequently dried and can be further processed to dosage forms.

In a preferred embodiment according to the invention pellets can be obtained by application of a preliminary isolation layer preferably by spraying a solution of hydroxypropyl methyl cellulose or polyvinylpyrrolidone onto sucrose starter pellets. Subsequently a suspension of com or maize starch as disintegration aid and the active compound in water (concentration of the active ingredient between 10 and 20%) and optionally other excipients can be applied.

In a preferred embodiment according to the invention an aqueous suspension of pregelatinized corn or maize starch and the active ingredient containing the binder (in dissolved form) and optionally containing other excipients is sprayed on starter pellets.

The spraying process is carried out according to methods known in the art, preferably in a fluidized bed apparatus (preferably in Wurster-modification) or in a conventional centrifugal coating pan.

The pellets according to the invention may be further processed to dosage forms, in particular oral dosage forms such as capsules or tablets or could be filled loose in primary packaging materials (e.g. aluminum pouches).

The active ingredient according to the invention is a pantoprazole magnesium salt having a low solubility in water, which allows providing aqueous suspensions of the active ingredient for the spraying process.

The average particle size of the suspended active ingredient (determined by suitable methods) is preferably in the range of 0.5 to 60 µm, particularly preferred in the range of 1.0 and 25 µm and most preferred between 1.0 and 15 5 µm. The active ingredient can be processed by suitable methods (e.g. milling or micronizing) to provide the desired particle size.

The active ingredient belongs to the group of acid-labile proton pump inhibitors, which are chiral compounds. The active ingredient includes the pure enantiomers and their mixtures in any mixing ratio.

For the first time, the international patent Application WO92/08716 describes a chemical process, which allows pyridin-2-ylmethylsulphinyl-1H-benzimidazoles to be separated into their optical antipodes. The compounds mentioned as being prepared in an exemplary manner include inter alia the compounds (+)- and (-)-5-difluoromethoxy-2-[(3,4-dimethoxy-2-pyridinyl)methylsulphinyl]1H-benzimidazole [= (+)-and (-)-pantoprazole]. The international patent application WO92/08716 mentions that the optical antipodes of the pyridin-2-ylmethylsulphinyl-1H-benzimidazoles, i.e. the (+)- and (-)-enantiomers or the (R)-and (S)-enantiomers, are used as active compounds in medicaments for the treatment of gastrointestinal disorders. For the mode of application and the dosage of the active compounds, reference is made inter alia to the European patent 166 287.

The international patent applications WO94/24867 and WO94/25028 claim the use of the compounds (-)- and (+)-pantoprazole for treating gastric disorders in humans- Each stereoisomer is said to have medical advantages compared to the respective other stereoisomers. The descriptions also mention a number of different possible salts of the stereoisomers, and particular preference is given to the sodium salt.

The International Patent Application WO97/41114 describes a specific process for the preparation of magnesium salts of pyridin-2-ylmethylsulfinyl-1H-benzimidazoles. Inter alia, the preparation of the magnesium salt of pantoprazole is also described by way of example. According to the analysis data indicated, the salt prepared is pantoprazole magnesium in anhydrous form.

International Patent Application WO00/10995 describes the dihydrate of the magnesium salt of pantoprazole. It is disclosed that the dihydrate of the magnesium salt of pantoprazole has inter alia improved stability properties as in comparison to pantoprazole itself or to pantoprazole sodium sesquihydrate.

International Patent Application WO04/013126 is related to (-)-pantoprazole magnesium and its hydrates and to medicaments comprising these compounds.

The active ingredients are present in the form of their salts with bases, i.e. as magnesium salts. If desired, said salts with bases can also be present in hydrate form. Such a hydrate of the salt of an acid-labile proton pump inhibitor with a base is disclosed, for example, in WO91/19710.

Particularly preferred pantoprazole magnesium salts, which, may be mentioned are (-)-pantoprazole magnesium, pantoprazole magnesium, (-)-pantoprazole magnesium dihydrate and pantoprazole magnesium dihydrate.

Pantoprazole is the INN (international Nonproprietary Name) for the compound 5-difluoromethoxy-2-[(3,4-dimethoxy-2-pyridinyl)methylsulfinyl]-1H-benzimidazole. The magnesium salt of pantoprazole is the chemical compound magnesium bis[5-[difluoromethoxy]-2-[[3,4-dimethoxy-2-pyridinyl]methyl]sulfinyl]-1H-benzimidazolide]. In connection with the invention the pantoprazole magnesium salt can also be present in hydrate form (e.g. monohydrate, sesquihadrate or dihydrate). A particular preferred hydrate in connection with the invention is the dihydrate of the magnesium salt of pantoprazole with the chemical name magnesium bis[5-[difluoromethoxy]-2-[[3,4-dimethoxy-2-pyridinyl]methyl]sulfinyl]-1H-benzimidazolide] dihydrate. The synthesis of the magnesium salt of pantoprazole is described for example in International Patent Application WO97/41114 and the synthesis of the dihydrate of the magnesium salt of pantoprazole is disclosed in International Patent Application WO00/10995.

Pantoprazole magnesium pellets, available as delayed release forms are found to have a prolonged and not complete in-vitro dissolution behaviour. By using (partially) pregelatinized starch (e.g. starch 15000®) in suspension together with other pharmaceutically acceptable excipients during the layering of seeds with the magnesium salt of pantoprazole the dissolution behaviour could be improved surprisingly. The increase in drug release from nonpareille pellets is evident after coating with a water-soluble intermediate layer and an enteric coating by processes.

Surprisingly it has also been found now that oral dosage forms for pantoprazole magnesium salt comprising pregelatinized corn or maize starch as disintegration aid in suspension show stability and a distinctly improved release profile for the active ingredient as compared to oral dosage forms for pantoprazole magnesium salt known from the art (see examples).

The invention therefore also relates to a dosage form for oral administration of pantoprazole magnesium salt comprising a capsule or a tablet containing the described nonpareille pellets in a therapeutically effective amount of the pantoprazole magnesium salt together with pregelatinized com or maize starch and one or more other suitable pharmaceutical excipients.

In connection with acid-labile proton pump inhibitors preferred dosage forms are multiparticulate forms such as pellets in a capsule or a multiple unit tableted dosage form (such as disclosed in WO 96/01623). with the dosage form advantageously being designed so that the pantoprazole magnesium salt is released, or made available effectively for the body, in such a way that an optimal active ingredient profile, and thus action profile, is achieved. Suitable dosage forms are for example disclosed in EP-A-0 519 365, EP-A-0 244 380, EP-A-1 213 015. EP-A-1 105 108. EP-A-1 037 634, EP-A-1 187 601 and EP-A-1 341 528.

In connection with acid-labile proton pump inhibitors the oral dosage form of the invention is preferably a dosage form with modified release of the active ingredient, in particular with delayed release of active ingredient. Particularly preferred is an enteric coated dosage form, comprising at least one enteric coating layer which is stable and does not release the active ingredient under acidic conditions but rapidly dissolves in neutral conditions and in particular in the alkaline medium of the intestine. In a further preferred embodiment the dosage form according to the invention in addition to the enteric coating layer contains one or more intermediate layers (subcoating layers). In another embodiment the dosage form according to the invention comprises at least one enteric coating layer but does not contain an intermediate layer.

Because of a great tendency to decompose in a neutral and, in particular, acidic environment, which also results in highly colored decomposition products, for oral compositions, it is preferred on the one hand to keep the proton pump inhibitor in an alkaline environment and, on the other hand, to protect it from exposure to acids. It is generally known to coat tablets or pellets, which contain an acid-labile active ingredient with an enteric coating which, after passage through the stomach, rapidly dissolves in the alkaline medium in the intestine. In the case of pantoprazole, which is very acid-labile, it is preferred to process it in the tablet core or in pellets in the form of its alkaline salts, and preferably together with alkaline substances. Since the substances suitable for enteric coatings contain free carboxyl groups, a problem arises when the enteric coating is partly or even completely dissolved from the inside because of the alkaline medium in the interior, and the free carboxyl groups promote decomposition of the active ingredients. It is therefore preferred to provide a sealing intermediate layer (subcoating) between the enteric coating and an alkaline tablet or pellet core. EP-A 0244380 proposes to coat cores, which contain the active ingredient together with alkaline compounds or as alkaline salt with at least one layer. which is soluble in water or rapidly disintegrates in water, of nonacidic, inert pharmaceutically acceptable substance before the enteric layer is applied.

The intermediate layer or intermediate layers act as pH-buffering zones in which hydrogen ions, which diffuse in from the outside, are able to react with the hydroxyl ions which diffuse out of the alkaline core. In order to increase the buffer capacity of the intermediate layer, it is proposed to incorporate buffer substance into the intermediate layer(s). It is possible in practice by this method to obtain rather stable compositions.

The invention therefore also relates to an oral dosage form of nonpareille pellets containing the active ingredient in a therapeutically effective amount together with pregelatinized com or maize starch and one or more other pharmaceutical excipients in an alkaline pellet core, at least one intermediate layer (subcoating) and an outer enteric layer (gastric resistant coating) which is soluble in the small intestine in the presence of neutral pH-values.

In another embodiment the invention also relates to an oral dosage form of nonpareille pellets containing the active ingredient in a therapeutically effective amount together with pregelatinized corn or maize starch and optionally one or more other pharmaceutical excipients in an alkaline pellet core, at least one intermediate layer (subcoating) and an outer enteric layer (gastric resistant coating) which is soluble in the small intestine in the presence of neutral pH-values.

In one embodiment of the invention the oral dosage form is a multiple unit tableted dosage form, with individual enteric coating layered units based on pellets according to the invention containing pantoprazole magnesium salt, pregelatinized com or maize starch - particularly pregelatinized starch - and optionally other excipients.

Further suitable pharmaceutical excipients, which may be used in the dosage forms forms for active ingredients according to this invention, are pharmaceutical excipients such as binders, disintegrants or else lubricants and release agents. Other suitable excipients, which may be present in the dosage form produced by the manufacturing process described in the invention are, for example, flavoring substances (such as flavors and sweeteners), buffer substances, preservatives, coloring substances (such as iron oxid yellow or red), wetting agents, surfactants (such as sodium laurylsulfate) or else emufsifiers. Flavors are usually added in a proportion of from 0.05 to 1% by weight Other flavoring substances by way of example are acids such as citric acid, sweeteners such as saccharin, aspartame, cyclamate sodium or maltol, which are added according to the desired result

For a basic reaction of the pellet core (= alkaline pellet core) it is mixed (where required increase in pH is not achieved simply by using an active-ingredient salt) with an inorganic base. Mention may be made in this connection of, for example, the pharmacologically-suitable (tolerable) alkali-metal, alkaline-earth-metal or earth-metal salts of weak acids and the pharmacologically-suitable hydroxides and oxides of alkaline-earth and earth metals. Sodium carbonate may be mentioned as an alkalic substance to be emphasized by way of example.

Besides binder, other ancillary substances, in particular lubricants and anti-sticking agents, and other disintegration aids, are used in the manufacture of the nonpareille pellet cores. Examples of lubricants and anti-sticking agents, which may be mentioned, are higher fatty acids and their alkali-metal and alkaline-earth-metal salts, such as calcium stearate. Another suitable lubricant is talc. Other suitable disintegration aids, in particular, chemically inert agents, which may be mentioned as preferred, are Crosslinked polyvinylpyrrolidone, crosslinked sodium carboxymethylcelluloses and sodium starch glycolate.

In one embodiment of the invention the oral dosage form according to the invention are nonpareille pellets comprising sodium carbonate, polyvinylpyrrolidone, sodium lauryl sulfate and (partially) pregelatinized starch as excipients for the pellet core layered together with the active ingredient on a sugar seed sphere. Preferably the active ingredient is pantoprazole magnesium dihydrate. Preferably the layer containing the active ingredient has a thickness of between 80 and 140 µm, in particular between 90 and 135 µm, 95 and 130 µm, 100 and 125 µm.

In respect of the intermediate layer(s) to be applied to a pellet core, reference may be made in particular to those water-soluble layers such as are usually used before application of layers which are resistant to gastric juice, or such as are described e.g. in DE-OS 39 01 151. Examples, which may be mentioned of film polymers, which can be used for the intermediate layer are hydroxypropylmethylcellulose and/or polyvinylpyrrolidone, to which plasticizers (such as, for example, propylene glycol) and/or other additives and auxiliaries (e.g. buffers, bases or pigments) can also be added if desired.

In one embodiment of the invention the oral dosage form according to the invention comprises intermediate layer(s) based on hydroxypropylmethylcellulose as film polymer.

The expert knows, on the basis of his technical knowledge, what outer layers, which are resistant to gastric juice can be used. Examples of suitable polymers for the enteric coating are methacrylic acid/methyl methacrylate copolymer or methacrylic acid/ethyl -acrylate copolymer (e.g. Eudragite® L. S, or Eudragit® L30D or a mixture of Eudragit® L30D and NE30D) or cellulose derivatives, such as carboxymethylethylcellulose (CMEC, Duodcel®), cellulose acetate phthalate (CAP), cellulose acetate trimellitate (CAT), hydroxypropylmethylcellulose phthalate (HP50, HPSS), hydroxypropylmethylcellulose acetate succinate (HPMCAS) or polyvinyl acetate phthalate, to which it is also possible to add, if desired, plasticizer (such as propylene glycol or triethyl citrate) and/or other additives and ancillary substances (e.g. surfactants, anti-sticking agents, buffers, bases, such as, preferably, aluminum hydroxide, or pigments).

In one embodiment of the invention the oral dosage form according to the invention comprises an enteric coating based on methacrylic acid/methyl methacrylate copolymer or methacrylic acid/ethylacrylate copolymer.

The layers are applied in conventional ways using equipment customary for these purposes.
The application of the different coatings on the pellet core can be carried out for example by processes known to the skilled worker for coating pellets (for example as disclosed in the various patent documents relating to oral dosage forms for proton pump inhibitors: the process mentioned in EP-A-0 519 365 or EP-A-0 244 380 may be mentioned by way of example).

The isolation layer or the enteric coating layer can also be applied on the pellets using corresponding ready-made dispersions (e.g. opadry, acryl-eeze) in a fluidized bed coater, preferably in Wurster modification.

Particularly preferred subject of the invention is therefore a manufacturing process to produce an oral dosage form in form of nonpareille pellets containing the magnesium salt of pantoprazole comprising the following steps:
(a) layering of an aqueous suspension of pregelatinized com or maize starch containing the active ingredient optionally together with other pharmaceutical excipients on starter pellets and
(b) coating of the obtained active pellets with water-soluble isolation layer and pH-dependent gastric resistant layer.

In a preferred embodiment of the above process pregelatinized starch and active ingredient are suspended in a solution of Kollidon K25 (molecular weight 28 000-34 000) before spraying on seed pellets.

In one embodiment the invention also relates to a dosage form or pharmaceutical product comprising pellets according to the invention contained in a primary packing material. Suitable primary packaging materials, which may be mentioned are foil sachets made of suitable foil material. Examples, which may be mentioned are four seamed foil sachets or three seamed foil sachets (which may also be referred to as stick pack). In this connection reference is also made to EP 0 705 204. Suitable foil materials, which may be mentioned are aluminium composite foils. Preferably an individual dose of pellets is contained in such a foil sachet. In order to reach an appropriate fill weight in connection with providing an individual dose of the active ingredient, placebo pellets (i.e. pellets not containing the active ingredient) may be added in a suitable ratio. Preferably placebo pellets are used which are comparable in size and colour to the pellets containing active ingredients. To this end suitable starter pellets (e.g. sucrose starter pellets) may be provided with an enteric coating, optionally containing an intermediate coating. Additionally the pellets may be mixed with a suitable glidant or anti-sticking agent to avoid sticking before filling in the foil sachet. Suitable glidants or anti-sticking agents, which may be mentioned are talc, magnesium stearate or com starch.

### Description of the figures

### Figures 1

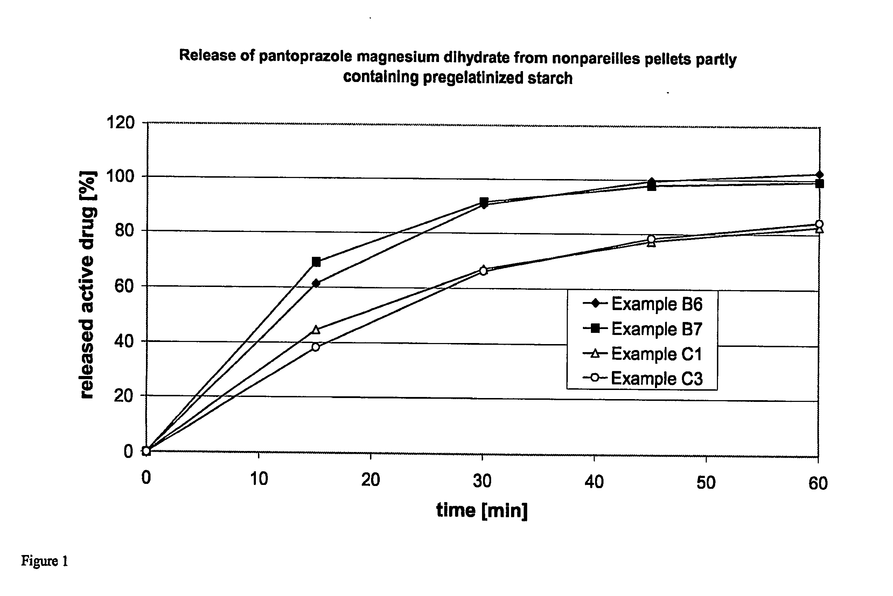

Figure 1 shows the release of the magnesium salt of pantoprazole from nonpareille pellets after isolation coating and enteric coating either containing pregelatinized starch in the pellet core or containing no pregelatinized starch. The pellets of the examples B1-B7 and C1-C3 were produced all by fluidized bed coating in Wurster modification.

The production of pellets and dosage forms according to the invention is described by way of example below. The following examples explain the invention in more detail without restricting **it.**

### Examples

### A. Synthesis of Magnesium bis[5-[difluoromethoxy]-2-[[3,4-dimethoxy-2-pyridinyl]-methyl]sulfinyl]-1H-benzimidazolide] dihydrate

3.85 kg (8.9 mol) of pantoprazole Na sesquihydrate [sodium [5-[difluoromethoxy]-2-[[3,4-dimethoxy-2-pyridinyl]methyl]sulfinyl]-1H-benzimidazolide]sesquihydrate] are dissolved at 20-25°C in 38.5 l of purified water in a stirring vessel. A solution of 1.0 kg (4.90 mol) of magnesium dichloride hexahydrate in 8 l of purified water is added with stirring at 20-30°C in the course of 3 to 4 h. After stirring for a further 18 h, the precipitated solid is centrifuged, washed with 23 l of purified water, stirred at 20-30°C for 1 to 2 h in 35 l of purified water, centrifuged again and washed again with 30-50 l of purified water. The solid product is dried at 50°C in vacuo (30-50 mbar) until a residual water content of < 4.8% is achieved. The product is then ground.

The title compound is obtained as a white to beige powder, which is employed directly for further pharmaceutical processing.

Yield: 3.40 kg (90% of theory); water content 4.5-4.6%; melting point 194-196°C with decomposition.

| CHN analysis | C | H | N | S |
|---|---|---|---|---|
| Theory | 46.58 | 3.91 | 10.19 | 7.77 |
| Found | 46.33 | 3.89 | 10.04 | 7.83 |

Alternatively the title compound can be produced using mixtures of organic solvents with water. For this, pantoprazole Na sesquihydrate is dissolved in an organic solvent at 50-60°C. 0.5 mole equivalents of the magnesium salt (e. g. magnesium chloride hexahydrate), dissolved in water, are added drop by drop and the solution is allowed to cool with stirring. The precipitated solid is filtered off, washed with the corresponding organic solvent and is dried in vacuo at 50°C to constant weight The title compound is obtained as a colourless powder. Examples for different solvents are given in the following table 1.

**Table 1:**

| pantoprazole Na sesquihydrate | organic solvent | water | yield of title compound | melting point °C | water content % |
|---|---|---|---|---|---|
| 50 g | isopropanol 300 ml | 300 ml | 45,4 g | 196-197 | 4,4-4,5 |
| 50 g | isopropanol 300 ml | 120 ml | 45,9 g | 196-197 | 4,3 |
| 50 g | ethanol 300 ml | 300 ml | 45,8 g | 197-198 | 4,6 |
| 50 g | aceton 300 ml | 300 ml | 45,6 g | 195-196 | 4,6, - 4.7 |

Alternatively the title compound can be produced by reacting pantoprazole with a basic magnesium salt, such as magnesium methylate, for example in the following manner: 90 g of pantoprazole are dissolved in 700 ml of 2-propanol at 60-70°C. 13.4 g (0.5 moles) of solid magnesium methylate are added, the solution is allowed to cool with stirring and filtered. After addition of 36 ml of water the crystalline solid formed is filtered off, washed with water and dried in vacuo at 50°C to constant weight. The title compound of melting point 194-196°C (water content 4.8 %) is obtained as beige solid.

### B. Production of dosage forms according to the invention

### Example B.1

Pellets made by Wurster coating (Nonpareilles):

### I. Active pellets:

| | |
|---|---|
| a.) Sucrose starter pellets (0.425-0.5 mm) | 500.0 g |
| b.) Sodium carbonate | 30.0 g |
| c.) Pregelatinized starch | 30.0 g |
| d.) Pantoprazole-Mg dihydrate | 300.0 g |
| e.) Polyvinylpyrrolidone K 25 | 35.0 g |

a. is sprayed with an aqueous suspension of b., c., d and e. in a fluidized bed process (Wurster equipment) or other suitable equipments (e.g. coating pan).

### II. Intermediate layer (subcoating):

| | |
|---|---|
| f.) Hydroxypropylmethylcellulose | 120.0 g |
| g.) Titanium dioxide | 20 g |
| h.) LB Iron oxide yellow | 0.2 g |
| i.) Propylene glycol | 24.0 g |

f. is dissolved in water (A). g. and h. are suspended in water using a high shear mixer (B). A and B are combined and after addition of i. the resulting suspension is sieved through a suitable sieve. The suspension is sprayed onto 500 g of the active pellets obtained under I using a fluidised bed process (Wurster) or other suitable processes (e.g. coating pan).

### III. Coating with a layer which is resistant to gastric juice (Enteric coating):

| | |
|---|---|
| j.) Eudragit® L 30 D | 230.0 g |
| k.) Triethyl citrate | 7.0 g |

j. is suspended in water and after addition of k. the resulting dispersion is sieved through a suitable sieve. lll is sprayed onto 500 g of the isolated pellets obtained under ll in a Wurster fluidised bed-apparatus or other suitable equipments (e.g. coating pan).

The resulting enteric coated pellets are mixed with talc (0.75%) and could be filled in hard gelatine capsules of suitable size (e.g. size 2) or tableted using suitable tableting ingredients (e.g. microcrystalline cellulose or lactose monohydrate) on a prevalent tablet press.

### Example B.2

Pellets made by Wurster coating (Nonpareilles):

### I. Active pellets:

| | |
|---|---|
| a.) Cellulose pellets (0.6-0.7 mm) | 1000.0 g |
| b.) Sodium carbonate | 75.0 g |
| c.) Pantoprazole-Mg dihydrate | 650.0 g |
| d.) Polyvinylpyrrolidone K 25 | 80.0 g |
| e.) Pregelatinized starch | 70.0 g |

a. is sprayed with an aqueous suspension of b., c., d. and e. in a fluidized bed process (Wurster equipment) or other suitable equipments (e.g. coating pan).

### ll. Intermediate layer (subcoating):

| | |
|---|---|
| f.) Hydroxypropylmethylcellulose | 250.0 g |
| g.) Titanium dioxide | 5.0 g |
| h.) LB Iron oxide yellow | 0.45 g |

f. is dissolved in water (A). g. and h. are suspended in water using a high shear mixer (B). A and B are combined and the resulting suspension is sieved through a suitable sieve. The suspension is sprayed onto 1000 g of the active pellets obtained under I using a fluidised bed process (Wurster) or other suitable processes (e.g. coating pan).

### III. Coating with a layer which is resistant to gastric juice (Enteric coating):

| | |
|---|---|
| i.) Eudragit® L 30 D | 365.0 g |
| j.) Triethyl citrate | 15.0 g |

i. is suspended in water and after addition of j. the resulting dispersion is sieved through a suitable sieve. III is sprayed onto 1000 g of the isolated pellets obtained under II in a Wurster fluidised bed-apparatus or other suitable equipments (e.g. coating pan).

The resulting enteric coated pellets are mixed with talc (0.5%) and could be filled in hard gelatine capsules of suitable size (e.g. size 2) or tableted using suitable tableting ingredients (e.g. microcrystalline cellulose or lactose monohydrate) on a prevalent tablet press.

### Example B.3

Pellets made by Wurster coating (Nonpareilles):

### I. Active pellets:

| | |
|---|---|
| a.) Cellulose pellets (0.4-0.5 mm) | 2000.0 g |
| b.) Sodium carbonate | 120.0 g |
| c.) (SrPantoprazole-Mg dihydrate | 1400.0 g |
| d.) Polyvinylpyrrolidone K 25 | 120.0 g |
| e.) Sodium dodecylsulfate (SDS) | 16.0 g |
| f.) Pregelatinized starch | 110.0 g |

To produce core material, suspension layering is performed in a fluid bed apparatus or other suitable equipment as described in example B1.

### II. intermediate layer (subcoating):

| | |
|---|---|
| g.) Hydroxypropylmethylcellulose | 600.0 g |
| h.) Polyvinylpyn-olidone K 25 | 8.0 g |
| i.) Titanium dioxide | 10.0 g |
| j.) LB Iron oxide yellow | 1.0 g |
| | |

The pellets covered with intermediate layer are produced as described in example B1.

### III. Coating with a layer which is resistant to gastric juice (Enteric coating):

| | |
|---|---|
| k.) Hydroxypropylmethylcellulose acetate succinate | 800.0 g |
| I.) Triethyl citrate | 250.0 g |
| m.) Ethanol | 7250.0 g |

The enteric coating layer is applied to the isolated pellets using fluidized bed equipment from a water/ethanol solution.

The resulting enteric coated pellets are mixed with talc and could be filled in hard gelatine capsules of suitable size (e.g. size 2) or tableted using suitable tableting ingredients (e.g. microcrystalline cellulose or lactose monohydrate) on a prevalent tablet press.

### Example B.4

Multiple unit tableted dosage form made from Nonpareille pellets:

### I. Active pellets:

| | |
|---|---|
| a.) Cellulose pellets (0.6-0.7 mm) | 2500.0 g |
| b.) Sodium carbonate | 180.0 g |
| c.) Pregelatinized starch | 160.0 g |
| d.) (S)-Pantoprazole-Mg dihydrate | 1700.0 g |
| e.) Polyvinylpyrrolidone K 25 | 250.0 g |
| f.) Sodium dodecylsulfate | 18.0 g |

a. is sprayed with an aqueous dispersion of b., c., d., e. and f. in a fluidized bed process (Wurster equipment) or other suitable equipments (e.g. coating pan).

### II. Intermediate layer (subcoating):

| | |
|---|---|
| g.) Hydroxypropylmethylcellulose | 600.0 g |
| h.) Talcum (micronized) | 100.0 g |
| i.) Magnesium stearate | 80.0g |

g. is dissolved in water (A). h. and i. are suspended in water using a high shear mixer (B). A and B are combined and the resulting suspension is sieved through a suitable sieve. The suspension is sprayed onto 2500 g of the active pellets obtained under I using a fluidised bed process (Wurster) or other suitable processes (e.g. coating pan).

### III. Coating with a layer which is resistant to gastric juice (Enteric coating):

| | |
|---|---|
| j.) Methacrylic acid copolymer | 925.0 g |
| k.) Polyethylene glycole 400 | 28.0 g |

j. is suspended in water and after addition of k. the resulting dispersion is sieved through a suitable sieve. III is sprayed onto 2500 g of the isolated pellets obtained under II in a Wurster fluidised bed-apparatus or other suitable equipments (e.g. coating pan).

### IV. Tablets:

| | |
|---|---|
| l.) Microcrystalline cellulose | 3750.0 g |
| m.) Crosslinked polyvinylpyrrolidone | 100.0 g |
| n.) Magnesium stearate | 7.0 g |

2500 g of enteric coated pellets are mixed with the tableting excipients and compressed into tablets using a single punch tableting machine equipped with 11 mm round punches. The dosage of pantoprazole is approx. 20 mg.

### Example B.5

Pellets made by Wurster coating (Nonpareilles):

### I. Active pellets:

| | |
|---|---|
| a.) Sucrose starter pellets (0.7 - 0.85 mm) | 4.0 kg |
| b.) Sodium carbonate | 0.27 kg |
| c.) Pantoprazole-Mg dihydrate | 2.84 kg |
| d.) Polyvinylpyrrolidone K 25 | 0.23 kg |
| e.) Pregelatinized starch | 0.22 kg |
| f.) Sodium dodecylsulfate | 0.03 kg |

a. is sprayed with an aqueous dispersion of the other ingredients in a fluidised bed process (Wurster equipment) or other suitable equipments (e.g. coating pan).

### II. Intermediate layer (subcoating):

| | |
|---|---|
| g.) Hydroxypropylmethylcellulose | 1.830 kg |
| h.) Titanium dioxide | 0.028 kg |
| i.) LB Iron oxide yellow | 0.003 kg |
| j.) Polyvinylpyrrolidone K25 | 0.021 kg |

g. and j. are dissolved in water (A). h. and i. are suspended in water using a high shear mixer (B). A and B are combined and the resulting suspension is sieved through a suitable sieve. The suspension is sprayed onto the active pellets obtained under I using a fluidised bed process (Wurster) or other suitable processes (e.g. coating pan).

### III. Coating with a layer which is resistant to gastric juice (Enteric coating):

| | |
|---|---|
| k.) Eudragit® L 30 D | 4.40 kg |
| I.) Triethyl citrate | 0.13 kg |
| m.) Talc | 0.06 kg |

k. is suspended in water and after addition of I. the resulting dispersion is sieved through a suitable sieve. The dispersion is sprayed on the isolated pellets obtained under II in a Wurster fluidised bed-apparatus or other suitable equipments (e.g. coating pan).

The resulting enteric coated pellets are mixed with talc (m) and could be filled in hard gelatine capsules of suitable size (e.g. size 2) or tableted using suitable tableting ingredients (e.g. microcrystalline cellulose or lactose monohydrate) on a prevalent tablet press.

### Example B.6

Pellets made by Wurster coating (Nonpareilles):

### I. Active pellets:

| | |
|---|---|
| a.) Sucrose starter pellets (0.7 - 0.85 mm) | 500.0 g |
| b.) Sodium carbonate | 32.0 g |
| c.) Pantoprazole-Mg dihydrate | 275.0 g |
| d.) Polyvinylpyrrolidone K 25 | 27.5 g |
| e.) Pregelatinized starch | 30.0 g |
| f.) Sodium dodecylsulfate | 4.5 g |

a. is sprayed with an aqueous dispersion of the other ingredients in a fluidised bed process (Wurster equipment) or other suitable equipments (e.g. coating pan).

### II. Intermediate layer (subcoating):

| | |
|---|---|
| g.) Hydroxypropylmethylcellulose | 108.0 g |
| h.) Titanium dioxide | 1.7 g |
| i.) LB Iron oxide yellow | 0.2 g |
| j.) Polyvinylpyrrolidone K25 | 1.3 g |

g. and j. are dissolved in water (A). h. and i. are suspended in water using a high shear mixer (B). A and B are combined and the resulting suspension is sieved through a suitable sieve. The suspension is sprayed onto 360.0 g of active pellets obtained under I using a fluidised bed process (Wurster) or other suitable processes (e.g. coating pan).

### III. Coating with a layer which is resistant to gastric juice (Enteric coating):

| | |
|---|---|
| k.) Eudragit® L 30 D | 436.0 g |
| I.) Triethyl citrate | 13.1 g |

k. is suspended in water and after addition of I. the resulting dispersion is sieved through a suitable sieve. The dispersion is sprayed on 400.0 g of the isolated pellets obtained under II in a Wurster fluidised bed-apparatus or other suitable equipments (e.g. coating pan).

The resulting enteric coated pellets are mixed with talc and could be filled in hard gelatine capsules of suitable size (e.g. size 2) or tableted using suitable tableting ingredients (e.g. microcrystalline cellulose or lactose monohydrate) on a prevalent tablet press.

### Example B.7

Pellets made by Wurster coating (Nonpareilles):

### I. Active pellets:

| | |
|---|---|
| a.) Sucrose starter pellets (0.7 - 0.85 mm) | 2000.0 g |
| b.) Sodium carbonate | 116.0 g |
| c.) Pantoprazole-Mg dihydrate | 1000.0 g |
| d.) Polyvinylpyrrolidone K 25 | 100.0 g |
| e.) Pregelatinized starch | 110.0 g |
| f.) Sodium dodecylsulfate | 16.4 g |

a. is sprayed with an aqueous dispersion of the other ingredients in a fluidised bed process (Wurster equipment) or other suitable equipments (e.g. coating pan).

### II. Intermediate layer (subcoating):

| | |
|---|---|
| g.) Hydroxypropylmethylcellulose | 902.8 g |
| h.) Titanium dioxide | 13.9 g |
| i.) LB Iron oxide yellow | 1.6 g |
| j.) Polyvinylpyrrolidone K25 | 10.4 g |

g. and j. are dissolved in water (A). h. and i. are suspended in water using a high shear mixer (B). A and B are combined and the resulting suspension is sieved through a suitable sieve. The suspension is sprayed onto 3000 g of active pellets obtained under I using a fluidised bed process (Wurster) or other suitable processes (e.g. coating pan).

### III. Coating with a layer which is resistant to gastric juice (Enteric coating):

| | |
|---|---|
| k.) Eudragit® L 30 D | 2024.0 g |
| l.) Triethyl citrate | 61.0 g |

k. is suspended in water and after addition of I. the resulting dispersion is sieved through a suitable sieve. The dispersion is sprayed on 3700 g of the isolated pellets obtained under II in a Wurster fluidised bed-apparatus or other suitable equipments (e.g. coating pan).

The resulting enteric coated pellets are mixed with talc and could be filled in hard gelatine capsules of suitable size (e.g. size 2) or tableted using suitable tableting ingredients (e.g. microcrystalline cellulose or lactose monohydrate) on a prevalent tablet press.

### Example B.8

Pellets according to example B.5 containing 40 mg pantoprazole are mixed with placebo pellets made by Wurster coating (Nonpareilles):

### Coating with a layer which is resistant to gastric juice (Enteric coating):

| | |
|---|---|
| a.) Sucrose starter pellets (0.85 -1.00 mm) | 4.000 kg |
| b.) Eudragit® L 30 D | 11.582 kg |
| c.) Titanium dioxide | 0.006 kg |
| d.) LB Iron oxide yellow | 0.002 kg |
| e.) Triethyl citrate | 0.346 kg |

c. and d. are suspended in water using a high shear mixer and added to the sieved suspension of b. e. is given to the resulting dispersion while stirring. The dispersion is sprayed on the sucrose starter pellets in a Wurster fluidised bed-apparatus or other suitable equipment (e.g. coating pan). The resulting particle size and the colour of the enteric coated placebo pellets are comparable to the pantoprazole pellets obtained under example B.5.

The resulting enteric coated placebo pellets are mixed with pellets obtained under example B.5 containing 40 mg Pantoprazole at a ratio of 2:1 to reach a fill weight of approx. 500 mg. Additionally, 0.5 % talc is added to the multiparticulates. The mixture is filled in a three-seamed tubular foil sachet of aluminium foil having a length of about 70 mm and a width of 23 mm.

### Example B.9

Pellets according to example B.5 containing 40 mg pantoprazole are mixed with placebo pellets made by Wurster coating (Nonpareilles):

### I. Intermediate layer (subcoating):

| | | |
|---|---|---|
| a.) | Sucrose starter pellets (0.85 -1.00 mm) | 4.000 kg |
| b.) | Hydroxypropylmethylcellulose | 0.963 kg |
| c.) | Titanium dioxid | 0.015 kg |
| d.) | LB Iron oxide yellow | 0.002 kg |
| e.) | Polyvinlypyn-olidone K25 | 0.011 kg |

b. and e. are dissolved in water (A). c. and d. are suspended in water using a high shear mixer (B). A and B are combined and the resulting suspension is sieved through a suitable sieve. The suspension is sprayed on the sucrose starter pellets using a fluidised bed process (Wurster) or other suitable processes (e.g. coating pan).

### II. Coating with a layer which is resistant to gastric juice (Enteric coating):

| | | |
|---|---|---|
| f.) | Eudragit L 30 D | 2.478 kg |
| g.) | Triethyl citrate | 0.074 kg |

f. is suspended in water and after addition of g. the resulting dispersion is sieved through a suitable sieve. The dispersion is sprayed on the isolated pellets obtained under I in a Wurster fluidised bed-apparatus or other suitable equipment (e.g. coating pan).

The resulting enteric coated placebo pellets are mixed with pellets obtained under example B.5 containing 40 mg pantoprazole at a ratio of 2:1 to reach a fill weight of approx. 500 mg. Additionally, 0.5 % talc is added to the multiparticulates. The mixture is filled in a three-seamed tubular foil sachet of aluminium foil having a length of about 70 mm and a width of 23 mm.

### Example B.10

The placebo pellets obtained under example B.8 are mixed with the pantoprazole pellets obtained under example B.5 containing 80 mg pantoprazole at a ratio 1:2 to reach a fill weight of approx. 500 mg. As lubricant 0.5 % talc is added to the multiparticulates. The mixture is filled in a three-seamed tubular foil sachet of aluminium foil having a length of about 70 mm and a width of 23 mm.

### C. Comparative tests with dosage forms in which no pregelatinized starch was used as disintegration aid:

### Example C.1

Pellets made by Wurster coating (Nonpareilles):

### I. Active pellets:

| | |
|---|---|
| a.) Sucrose starter pellets (0.7 - 0.85 mm) | 2000.0 g |
| b.) Sodium carbonate | 128.3 g |
| c.) Pantoprazole-Mg dihydrate | 1350.0 g |
| d.) Polyvinylpyrrolidone K 25 | 120.0 g |
| e.) Sodium dodecylsulfate | 18.3 g |

a. is sprayed with an aqueous dispersion of the other ingredients in a fluidised bed process (Wurster equipment) or other suitable equipments (e.g. coating pan).

### II. Interrmediate layer (subcoating):

| | |
|---|---|
| f.) Hydroxypropylmethylcellulose | 88.0 g |
| g.) Titanium dioxide | 2.0 g |
| h.) LB Iron oxide yellow | 0.2 g |
| i.) Polyvinylpyrrolidone K25 | 37.0 g |

f. and i. are dissolved in water (A). g. and h. are suspended in water using a high shear mixer (B). A and B are combined and the resulting suspension is sieved through a suitable sieve. The suspension is sprayed onto 400 g of active pellets obtained under I using a fluidised bed process (Wurster) or other suitable processes (e.g. coating pan).

### III. Coating with a layer which is resistant to gastric juice (Enteric coating):

| | |
|---|---|
| j.) Eudragit® L 30 D | 218.0 g |
| k.) Triethyl citrate | 6.5 g |

j. is suspended in water and after addition of k. the resulting dispersion is sieved through a suitable sieve. The dispersion is sprayed on 400.0 g of the isolated pellets obtained under II in a Wurster fluidised bed-apparatus or other suitable equipments (e.g. coating pan).

The resulting enteric coated pellets are mixed with talc and could be filled in hard gelatine capsules of suitable size (e.g. size 2) or tableted using suitable tableting ingredients (e.g. microcrystalline cellulose or lactose monohydrate) on a prevalent tablet press.

### Example C.2

Pellets made by Wurster coating (Nonpareilles):

### I. Active pellets:

| | |
|---|---|
| a:) Sucrose starter pellets (0.5 - 0.6 mm) | 2000.0 g |
| b.) Sodium carbonate | 128.3 g |
| c.) Pantoprazole-Mg dihydrate | 1350.0 g |
| d.) Polyvinylpyrrolidone K 25 | 130.0 g |
| e.) Sodium dodecylsulfate | 18.3 g |

a. is sprayed with an aqueous dispersion of the other ingredients in a fluidised bed process (Wurster equipment) or other suitable equipments (e.g. coating pan).

### II. Intermediate layer (subcoating):

| | |
|---|---|
| f.) Hydroxypropylmethylcellulose | 8722 g |
| g.) Titanium dioxide | 13.7 g |
| h.) LB Iron oxide yellow | 1.5 g |
| i.) Polyvinylpyrrolidone K25 | 10.2 g |

f. and i. are dissolved in water (A). g. and h. are suspended in water using a high shear mixer (B). A and B are combined and the resulting suspension is sieved through a suitable sieve. The suspension is sprayed onto 3000 g of active pellets obtained under I using a fluidised bed process (Wurster) or other suitable processes (e.g. coating pan).

### III. Coating with a layer which is resistant to gastric juice (Enteric coating):

| | |
|---|---|
| j.) Eudragit® L 30 D | 1730.0 g |
| k.) Triethyl citrate | 54.0 g |

j. is suspended in water and after addition of k. the resulting dispersion is sieved through a suitable sieve. The dispersion is sprayed on 3130 g of the isolated pellets obtained under II in a Wurster fluidised bed-apparatus or other suitable equipments (e.g. coating pan).

The resulting enteric coated pellets are mixed with talc and could be filled in hard gelatine capsules of suitable size (e.g. size 2) or tableted using suitable tableting ingredients (e.g. microcrystalline cellulose or lactose monohydrate) on a prevalent tablet press.

### Example C.3

Pellets made by Wurster coating (Nonpareilles):

### I. Active pellets:

| | |
|---|---|
| a.) Sucrose starter pellets (0.7 - 0.85 mm) | 2000.0 g |
| b.) Sodium carbonate | 115.5 g |
| c.) Pantoprazole-Mg dihydrate | 1215.0 g |
| d.) Polyvinylpyrrolidone K 25 | 117.0 g |
| e.) Sodium dodecylsulfate | 18.3 g |

a. is sprayed with an aqueous dispersion of the other ingredients in a fluidised bed process (Wurster equipment) or other suitable equipments (e.g. coating pan).

### II. Intermediate layer (subcoating):

| | |
|---|---|
| f.) Hydroxypropylmethylcellulose | 88.0 g |
| g.) Titanium dioxide | 2.0 g |
| h.) LB Iron oxide yellow | 0.2 g |
| i.) Polyvinylpyrrolidone K25 | 37.0 g |

f. and i. are dissolved in water (A). g. and h. are suspended in water using a high shear mixer (B). A and B are combined and the resulting suspension is sieved through a suitable sieve. The suspension is sprayed onto 400 g of active pellets obtained under I using a fluidised bed process (Wurster) or other suitable processes (e.g. coating pan).

### III. Coating with a layer which is resistant to gastric juice (Enteric coating):

| | |
|---|---|
| j.) Eudragit® L 30 D | 218.0 g |
| k.) Triethyl citrate | 6.5 g |

j. is suspended in water and after addition of k. the resulting dispersion is sieved through a suitable sieve. The dispersion is sprayed on 400.0 g of the isolated pellets obtained under II in a Wurster fluidised bed-apparatus or other suitable equipments (e.g. coating pan).

The resulting enteric coated pellets are mixed with talc and could be filled in hard gelatine capsules of suitable size (e.g. size 2) or tableted using suitable tableting ingredients (e.g. microcrystalline cellulose or lactose monohydrate) on a prevalent tablet press.

### Release of active Ingredient:

The release of the active ingredient was determined as described in the US Pharmacopoeia (USP XXV; apparatus 2; 2 hours 0.1 N HCI and 1 hour phosphate buffer pH 6.8; 100 rpm). Figure 1 shows the drug release after 1 hour in phosphate buffer pH 6.8. As can be seen, the examples according to the invention (example B6 and B7) show a faster release of active drug.

### D. Administration of dosage forms according to the invention

### Example D.1

The sachet obtained under example B.8, B.9 or B.10 is opened and the content filled into a syringe barrel. Apple juice is added into the syringe barrel to suspend the pellets. The resulting suspension is administered to a patient through a nasogastric tube with the size Charriere 14.

### Example D.2

The multiparticulate mixture obtained under example B.8, B.9 or B.10 is mixed into a drink, e.g. apple juice, for consumption by a patient.

### Example D.3

The sachet obtained under example B.8 , B.9 or B.10 is emptied and the multiparticulates are sprinkled on a food product, e.g. applesauce, for consumption by a patient.

### Industrial applicability

The manufacturing process according to the invention - spraying of a suspension of pregelatinized starch together with other suitable excipients on seed pellets in a fluidized bad apparatus - can be used for economic and feasible production of nonpareille pellets containing a pantoprazole magnesium salt as an active ingredient. Such dosage forms may be used for the treatment of diseases which are regarded as treatable or avoidable by the use of the particular active ingredient.

The dosage forms according to the invention containing the magnesium salt of pantopr-azole can be employed for the treatment and prevention of all the diseases, which are regarded as treatable or avoidable by the use of pyridin-2-ylmethylsulfinyl-1H-benzimidazoles. In particular, such dosage forms according to the invention can be employed in the treatment of stomach disorders. Examples which may be mentioned in connection with the invention are the treatment or prophylaxis of benign gastric ulcer, gastro-oesophageal reflux disease, Zollinger-Ellison syndrome, duodenal ulcer, duodenal ulcer associated with Helicobacter pylori, prophylaxis of NSAID-associated gastric or duodenal ulcer in patients with an increased risk of gastroduodenal complication who require continued NSAID treatment or combination therapy with antibiotics in the eradication of Helicobacter pylori. Such dosage forms according to the invention contain between 1 and 500 mg, preferably between 5 and 100 mg, particularly preferable between 5 and 80 mg of the pantoprazole. Examples which may be mentioned are tablets, capsules or foil sachets which contain the pantoprazole magnesium salt in an amount corresponding to 10, 20, 40, 50, 80 or 100 mg of pantoprazole (free acid). The administration of the daily dose (e.g. 40 mg of active compound) can be carried out, for example, in the form of an individual dose or by means of a number of doses of the administration forms according to the invention (e.g. 2 times 20 mg of active compound). In connection with the pharmaceutical product containing the pellets according to the invention the pellets may be suspended in a suitable carrier prior to administration. Suitable carriers, which may be mentioned are physiologically acceptable carriers, preferably having a pH below 7, preferably below 6, in particular preferably below 5.5 in order to avoid dissolution of the enteric coating. Physiologically acceptable carriers are for example fruit juices such as apple juice or orange juice or buffer solutions. Other suitable carriers are food products such as apple sauce. In one embodiment according to the invention a suspension of the pellets according to the invention in a suitable liquid (such as apple juice) may also be administered through a nasogastric tube. To this end the suspension is provided in a syringe and subsequently administered throught the nasogastric tube. This way of administration is particular suitable for pediatric patients or patients having difficulties in swallowing solid oral formulations.

The invention therefore also relates to a method for the prophylaxis or treatment of a clinical condition in a mammal, such as a human, for which a proton pump inhibitor is indicated, which comprises administration of a therapeutically effective amount pantoprazole magnesium in a dosage form according to the invention. In one embodiment the clinical condition is selected from the group of benign gastric ulcer, gastro-oesophageal reflux disease, Zollinger-Ellison syndrome, duodenal ulcer, duodenal ulcer associated with helicobacter pylori, prophylaxis of NSAID-associated gastric or duodenal ulcer in patients with an increased risk of gastroduodenal complication who require continued NSAID treatment and combination therapy with antibiotics in the eradication of Helicobacter pylori.

The dosage forms according to the invention can be combined with other medicaments, either in various combinations or in a fixed combination. In connection with the administration forms according to the invention, which contain magnesium salt of pantoprazole as active compounds, combinations with antimicrobial active compounds and combinations with NSAIDs (nonsteroidal antiinflammatory drugs) are particularly worthy of mention. Combination with antimicrobial agents, such as are employed for the control of the microorganism Helicobacter pylori (H. pylori), may particularly be mentioned.

Examples of suitable antimicrobial active compounds (active against Helicobacter pylon) are described in EP-A-0 282 131. Examples of antimicrobial agents suitable for the control of the microorganism Helicobacter pylori which may be mentioned are, for example, bismuth salts [e.g. bismuth subcitrate, bismuth subsalicylate, ammonium bismuth(III) potassium citrate dihydroxide, bismuth nitrate oxide, dibismuth tris(tetraoxodialuminate)], but in particular β-lactam antibiotics, for example penicillins (such as benzylpenicillin, phenoxymethylpenicillin, propicillin, azidocillin, dicloxacillin, fludoxacillin, oxacillin, amoxicillin, bacampicillin, ampicillin, mezlocillin, piperacillin or azlocillin), cephalosporins (such as cefadroxil, cefaclor, cefalexin, cefixime, cefuroxime, cefetamet, cefadroxil, ceftibuten, cefpodoxime, cefotetan, cefazolin, cefoperazon, ceftizoxime, cefotaxime, ceftazidime, cefamandol, cefepime, cefoxitin, cefodizime, cefsulodin, ceftriaxon, cefotiam or cefmenoxime) or other β-lactam antibiotics (e.g. aztreonam, loracarbef or meropenem); enzyme inhibitors, for example sulbactem; tetracyclines, for example tetracycline, oxytetracycline, minocycline or doxycycline; aminoglycosides, for example tobramycin, gentamicin, neomycin, streptomycin, amikacin, netilmicin, paromomycin or spectinomycin; amphenicols, for example chloramphenicol or thiamphenicol; lincomycins and macrolide antibiotics, for example clindamycin, lincomycin, erythromycin, clarithromycin, spiramycin, roxithromycin or azithromycin, polypeptide antibiotics, for example colistin, polymixin B, teicoplanin or vancomycin; gyrase inhibitors, for example norfloxacin, cinoxacin, ciprofloxacin, pipemidic acid, enoxacin, nalidixic acid, pefloxacin, fleroxacin or ofloxacin; nitroimidazoles, for example metronidazole; or other antibiotics, for example fosfomycin or fusidic acid. particularly worthy of mention in this connection is the administration of the magnesium salt of pantoprazole with the combination of a multiplicity of antimicrobial active compounds, for example with the combination of a bismuth salt and/or tetracyclines with metronidazole or the combination of amoxicillin or clarithromycin with metronidazole and amoxicillin with clarithromycin.

## Claims

1. Pellet comprising a starter pellet layered with a layer comprising an active ingredient, disintegrant and optionally other pharmaceutically acceptable excipients, wherein the layer is formed by spraying a suspension of the disintegrant containing the active ingredient and optionally other pharmaceutically active excipients onto the starter pellet, whereby the disintegrant is pregelatinized corn or maize starch and said active ingredient is a pantoprazole magnesium salt.

2. Pellet according to claim 1, wherein the starter pellet is based on materials selected from the group of cellulose, sucrose, starch and hydroxypropyl methyl cellulose.

3. Pellet according to claim 1, wherein the particle size of the starter pellets is in the range of 0.25 and 1.4 mm, preferably between 0.4 and 1.3 mm and most particularly preferred in the range of 0.6 and 1.0 mm.

4. Pellet according to claim 1, wherein the starch is partially pregelatinized starch.

5. Pellet according to claim 1, wherein the quantity (in percent of weight based on the active pellet core without further optional coatings) of pregelatinized maize or corn starch is in the range of 0.5 and 5%, in the range of 1.0 and 4.0% or in the range of 2.0 and 3.5%.

6. Pellet according to claim 1, wherein the pantoprazole magnesium salt is selected from the group of (-)-pantoprazole magnesium, pantoprazole magnesium, (-)-pantoprazole magnesium dihydrate and pantoprazole magnesium dihydrate.

7. Pellet according to claim 1, wherein polyvinylpyrrolidone and/or hydroxypropylmethylcellulose is present as binder.

8. Pellet according to claim 1, wherein a basic, physiologically tolerated inorganic compound is present.

9. Pellet according to claim 8, wherein pharmacologically tolerated alkali metal, alkaline earth metal or earth metal salt of a weak acid or pharmacologically tolerated hydroxide or oxide of an alkaline earth or earth metal is the basic, physiologically tolerated inorganic compound.

10. Pellet according to claim 9, wherein sodium carbonate is the basic, physiologically tolerated inorganic compound

11. Oral dosage form comprising a pantoprazole magnesium salt together with one or more pharmaceutically acceptable excipients comprising pellets according to any of the preceeding claims.

12. Oral dosage form according to claim 11, which dosage form is selected from the group of capsules or tablets or pellets filled loose in primary packaging materials.

13. Dosage form according to claim 11, which is a solid dosage form in nonpareille pellets form.

14. Dosage form according to claim 11, which is a delayed release dosage form comprising an enteric layer, which is soluble in neutral or alkaline conditions and at least one intermediate layer (subcoating layer).

15. Dosage form according to claim 11, wherein the pantoprazole magnesium dihydrate or (-)-pantoprazole magnesium dihydrate are present as active ingredient.

16. Dosage form according to claim 15 in pellet form, comprising a pellet core, an intermediate layer and an enteric coating, wherein the pellet core is formed from sucrose starter pellets, active ingredient, starch and optionally other excipients.

17. Dosage form according to claim 16, wherein the starch is pregelatinized starch.

18. Dosage form according to claim 17, wherein the pregelatinized starch is (partially) pregelatinized corn starch.

19. Dosage form according to claim 11, comprising a pellet core an intermediate layer and an enteric coating, wherein the pellet core is formed from sucrose starter pellets, pantoprazole magnesium dihydrate or (-)-pantoprazole magnesium dihydrate, sodium carbonate, PVP 25, pregelatinized starch and sodium dodecylsulfate, the intermediate layer is formed of HPMC, PVP 25, titanium dioxide and iron oxide yellow, and the enteric coating is formed of Eudragit L 30 D and triethyl citrate.

20. Dosage form according to claim 19, wherein the sucrose starter pellets are layered with a layer of pantoprazole magnesium dihydrate or (-)-pantoprazole magnesium dihydrate, sodium carbonate, PVP 25, pregelatinized starch and sodium dodecylsulfate.

21. Dosage form according to claim 11, containing between 5 and 100 mg, of the magnesium salt of pantoprazole.

22. Dosage form according to claim 21, which contain an amount of the magnesium salt of pantoprazole, which corresponds to 10, 20, 40, 50, 80 or 100 mg of pantoprazole (free acid).

23. Dosage form according to claim 21, which contain an amount of the magnesium salt of pantoprazole, which corresponds to 40 mg of pantoprazole (free acid).

24. Process for manufacturing a pellet according to claim 1 by spraying a suspension of the disintegrant containing the active ingredient optionally other excipients on starter pellets and drying the pellets, whereby the disintegrant is pregelatinized maize or corn starch.

25. Process according to claim 24, wherein the pregelatinized starch is pregelatinized corn starch.

26. Process according to any of claims 24 and 25, wherein the suspension is an aqueous suspension of the disintegrant and the active ingredient.

27. Process for manufacturing a dosage form according to claim 11 by spraying a aqueous suspension of pregelatinized starch additionally containing magnesium salt of pantoprazole, sodium carbonate, sodium dodecylsulfate and PVP as binder on starter pellets, drying the pellets, layering them with subcoating and enteric coating, mixing with glidants where applicable and filling into capsules.

28. Process according to claim 27, wherein the enteric coating is added after repeated drying after layering with the subcoating.

29. Process according to claim 28, which is carried out in a fluidized bed apparatus.

30. Process for manufacturing a dosage form according to claim 11 by spraying a aqueous suspension of pregelatinized starch additionally containing magnesium salt of pantoprazole, sodium carbonate, sodium dodecylsulfate and PVP as binder on starter pellets, drying the pellets, layering them with subcoating and enteric coating, mixing with placebo pellets and glidants where applicable and filling into foil sachets.

31. Dosage form according to claim 20, wherein the layer has a thickness of between 80 and 140 µm, 90 and 135 µm, 95 and 130 µm, 100 and 125 µm.

## Patentansprüche

1. Pellet, umfassend ein Starterpellet, das mit einer Schicht, umfassend einen Wirkstoff, Sprengmittel und wahlweise andere pharmazeutisch verträgliche Hilfsstoffe, beschichtet ist, wobei die Schicht durch Sprühen einer Suspension des Sprengmittels, enthaltend den Wirkstoff und wahlweise andere pharmazeutisch wirksame Hilfsstoffe, auf das Starterpellet gebildet ist, wobei das Sprengmittel vorgelatinierte Speise- oder Maisstärke ist, und der genannte Wirkstoff ein Pantoprazol-Magnesiumsalz ist.

2. Pellet gemäß Anspruch 1, wobei das Starterpellet auf Materialien ausgewählt aus der Gruppe von Cellulose, Sucrose, Stärke und Hydroxypropylmethylcellulose basiert.

3. Pellet gemäß Anspruch 1, wobei die Teilchengröße der Starterpellets im Bereich von 0,25 bis 1,4 mm liegt, vorzugsweise zwischen 0,4 und 1,3 mm, höchst bevorzugt im Bereich von 0,6 bis 1,0 mm.

4. Pellet gemäß Anspruch 1, wobei die Stärke teilweise vorgelatinierte Stärke ist.

5. Pellet gemäß Anspruch 1, wobei die Menge (in Gewichtsprozent, bezogen auf den aktiven Pelletkern ohne weitere optionale Beschichtungen) an vorgelatinierter Mais- oder Speisestärke im Bereich von 0,5 bis 5 %, im Bereich von 1,0 bis 4,0 % oder im Bereich von 2,0 bis 3,5 % liegt.

6. Pellet gemäß Anspruch 1, wobei das Pantoprazol-Magnesiumsalz ausgewählt ist aus der Gruppe von (-)-Pantoprazol-Magnesium, Pantoprazol-Magnesium, (-)-Pantoprazol-Magnesiumdihydrat und Pantoprazol-Magnesiumdihydrat.

7. Pellet gemäß Anspruch 1, wobei Polyvinylpyrrolidon und/oder Hydroxypropylmethylcellulose als Bindemittel vorhanden ist.

8. Pellet gemäß Anspruch 1, wobei eine basische, physiologisch tolerierte anorganische Verbindung vorhanden ist.

9. Pellet gemäß Anspruch 8, wobei die basische, physiologisch tolerierte anorganische Verbindung pharmakologisch toleriertes Alkalimetall-, Erdalkalimetall- oder Erdmetallsalz einer schwachen Säure oder pharmakologisch toleriertes Hydroxid oder Oxid eines Erdalkali- oder Erdmetalls ist.

10. Pellet gemäß Anspruch 9, wobei die basische, physiologisch tolerierte anorganische Verbindung Natriumcarbonat ist.

11. Orale Darreichungsform, umfassend ein Pantoprazol-Magnesiumsalz zusammen mit einem oder mehreren pharmazeutisch verträglichen Hilfsstoffen, umfassend Pellets gemäß einem der vorhergehenden Ansprüchen.

12. Orale Darreichungsform gemäß Anspruch 11, wobei die Darreichungsform ausgewählt ist aus der Gruppe von Kapseln oder Tabletten oder Pellets, die lose in primäre Verpackungsmaterialien gefüllt sind.

13. Darreichungsform gemäß Anspruch 11, die eine feste Darreichungsform in der Form von Nonpareille-Pellets darstellt.

14. Darreichungsform gemäß Anspruch 11, die eine Darreichungsform mit verzögerter Freisetzung ist, umfassend einen magensaftresistenten Überzug, der bei neutralen oder alkalischen Bedingungen löslich ist, und wenigstens eine Zwischenschicht (Unterschicht).

15. Darreichungsform gemäß Anspruch 11, wobei Pantoprazol-Magnesiumdihydrat oder (-)-Pantoprazol-Magnesiumdihydrat als Wirkstoff vorhanden ist.

16. Darreichungsform gemäß Anspruch 15 in Pelletform, umfassend einen Pelletkern, eine Zwischenschicht und einen magensaftresistenten Überzug, wobei der Pelletkern aus Sucrose-Starterpellets, Wirkstoff, Stärke und wahlweise anderen Hilfsstoffen gebildet ist.

17. Darreichungsform gemäß Anspruch 16, wobei die Stärke vorgelatinierte Stärke ist.

18. Darreichungsform gemäß Anspruch 17, wobei die vorgelatinierte Stärke (teilweise) vorgelatinierte Speisestärke ist.

19. Darreichungsform gemäß Anspruch 11, umfassend einen Pelletkern, eine Zwischenschicht und einen magensaftresistenten Überzug, wobei der Pelletkern aus Sucrose-Starterpellets, Pantoprazol-Magnesiumdihydrat oder (-)-Pantoprazol-Magnesiumdihydrat, Natriumcarbonat, PVP 25, vorgelatinierter Stärke und Natriumdodecylsulfat gebildet ist, die Zwischenschicht aus HPMC, PVP 25, Titandioxid und Eisenoxidgelb gebildet ist und der magensaftresistente Überzug aus Eudragit L 30 D und Triethylcitrat gebildet ist.

20. Darreichungsform gemäß Anspruch 19, wobei die Sucrose-Starterpellets mit einer Schicht aus Pantoprazol-Magnesiumdihydrat oder (-)-Pantoprazol-Magnesiumdihydrat, Natriumcarbonat, PVP 25, vorgelatinierter Stärke und Natriumdodecylsulfat beschichtet sind.

21. Darreichungsform gemäß Anspruch 11, enthaltend zwischen 5 und 100 mg des Magnesiumsalzes von Pantoprazol.

22. Darreichungsform gemäß Anspruch 21, welche eine Menge des Magnesiumsalzes von Pantoprazol enthält, die 10, 20, 40, 50, 80 oder 100 mg Pantoprazol (freie Säure) entspricht.

23. Darreichungsform gemäß Anspruch 21, welche eine Menge des Magnesiumsalzes von Pantoprazol enthält, die 40 mg Pantoprazol (freie Säure) entspricht.

24. Verfahren zum Herstellen eines Pellets gemäß Anspruch 1 durch Sprühen einer Suspension des Sprengmittels, enthaltend den Wirkstoff und wahlweise andere Hilfsstoffe, auf Starterpellets und Trocknen der Pellets, wobei das Sprengmittel vorgelatinierte Mais- oder Speisestärke ist.

25. Verfahren gemäß Anspruch 24, wobei die vorgelatinierte Stärke vorgelatinierte Speisestärke ist.

26. Verfahren gemäß einem der Ansprüche 24 und 25, wobei die Suspension eine wässrige Suspension des Sprengmittels und des Wirkstoffs ist.

27. Verfahren zum Herstellen einer Darreichungsform gemäß Anspruch 11 durch Sprühen einer wässrigen Suspension aus vorgelatinierter Stärke, zusätzlich enthaltend Magnesiumsalz von Pantoprazol, Natriumcarbonat, Natriumdodecylsulfat und PVP als Bindemittel auf Starterpellets, Trocknen der Pellets, deren Beschichten mit Unterbeschichtung und magensaftresistentem Überzug, gegebenenfalls Mischen mit Gleitmitteln und Abfüllen in Kapseln.

28. Verfahren gemäß Anspruch 27, wobei der magensaftresistente Überzug nach wiederholtem Trocknen nach Beschichten mit der Unterbeschichtung zugegeben wird.

29. Verfahren gemäß Anspruch 28, das in einer Wirbelschichtvorrichtung durchgeführt wird.

30. Verfahren zum Herstellen einer Darreichungsform gemäß Anspruch 11 durch Sprühen einer wässrigen Suspension aus vorgelatinierter Stärke, zusätzlich enthaltend Magnesiumsalz von Pantoprazol, Natriumcarbonat, Natriumdodecylsulfat und PVP als Bindemittel, auf Starterpellets, Trocknen der Pellets, deren Beschichten mit Unterbeschichtung und magensaftresistentem Überzug, gegebenenfalls Mischen mit Placebopellets und Gleitmitteln und Abfüllen in Folienpackungen.

31. Darreichungsform gemäß Anspruch 20, wobei die Schicht eine Dicke zwischen 80 und 140 µm, 90 und 135 µm, 95 und 130 µm, 100 und 125 µm aufweist.

## Revendications

1. Pastille comprenant une pastille de départ enduite avec une couche comprenant une substance active, un délitant et facultativement d'autres excipients pharmaceutiquement acceptables, où la couche est formée par pulvérisation d'une suspension du délitant contenant la substance active et facultativement d'autres excipients pharmaceutiquement actifs sur la pastille de départ, où le délitant est du maïs prégélatinisé ou de l'amidon de maïs et ladite substance active est un sel de magnésium de pantoprazole.

2. Pastille selon la revendication 1, où la pastille de départ est à base de matériaux choisis dans le groupe de la cellulose, le saccharose, l'amidon et l'hydroxypropylméthylcellulose.

3. Pastille selon la revendication 1, où la taille de particule des pastilles de départ est dans la plage de 0,25 et 1,4 mm, de préférence entre 0,4 et 1,3 mm et de manière préférée entre toutes dans la plage de 0,6 à 1,0 mm.

4. Pastille selon la revendication 1, où l'amidon est de l'amidon partiellement prégélatinisé.

5. Pastille selon la revendication 1, où la quantité (en pour cent en poids sur la base du noyau de pastille actif sans autres enrobages éventuels) de maïs prégélatinisé ou d' amidon de maïs est dans la plage de 0,5 à 5 %, dans la plage de 1,0 à 4,0 % ou dans la plage de 2,0 et 3,5 %.

6. Pastille selon la revendication 1, où le sel de magnésium de pantoprazole est choisi dans le groupe de (-)-pantoprazole magnésium, pantoprazole magnésium, (-)-pantoprazole magnésium dihydraté et pantoprazole magnésium dihydraté.

7. Pastille selon la revendication 1, où la polyvinylpyrrolidone et/ou l'hydroxypropylméthylcellulose est présente en tant que liant.

8. Pastille selon la revendication 1, où un composé inorganique basique, physiologiquement toléré est présent.

9. Pastille selon la revendication 8, dans laquelle un métal alcalin, un métal alcalino-terreux ou un sel de métal alcalino-terreux pharmacologiquement toléré d'un acide faible ou un hydroxyde ou oxyde pharmacologiquement toléré d'un métal alcalino-terreux ou alcalin est le composé inorganique basique, physiologiquement toléré.

10. Pastille selon la revendication 9, où le carbonate de sodium est le composé inorganique basique, physiologiquement toléré.

11. Forme pharmaceutique orale comprenant un sel de magnésium de pantoprazole conjointement avec un ou plusieurs excipients pharmaceutiquement acceptables comprenant des pastilles selon l'une quelconque des revendications précédentes.

12. Forme pharmaceutique orale selon la revendication 11, ladite forme pharmaceutique est choisie dans le groupe de capsules ou comprimés ou pastilles remplis librement dans des matériaux de conditionnement primaire.

13. Forme pharmaceutique selon la revendication 11, qui est une forme pharmaceutique solide sous forme de pastilles nonpareilles.

14. Forme pharmaceutique selon la revendication 11, qui est une forme pharmaceutique à libération retardée comprenant une couche entérique, qui est soluble dans des conditions neutres ou alcalines et au moins une couche intermédiaire (couche de sous-enrobage).

15. Forme pharmaceutique selon la revendication 11, dans laquelle le pantoprazole magnésium dihydraté ou le (-)-pantoprazole magnésium dihydraté sont présents en tant que substance active.

16. Forme pharmaceutique selon la revendication 15 sous forme de pastille, comprenant un noyau de pastille, une couche intermédiaire et un enrobage entérique, où le noyau de pastille est formé de pastilles de départ de saccharose, de substance active, d'amidon et facultativement d'autres excipients.

17. Forme pharmaceutique selon la revendication 16, dans laquelle l'amidon est de l'amidon prégélatinisé.

18. Forme pharmaceutique selon la revendication 17, dans laquelle l'amidon amidon prégélatinisé est de l'amidon de maïs (partiellement) prégélatinisé.

19. Forme pharmaceutique selon la revendication 11, comprenant un noyau de pastille, une couche intermédiaire et un enrobage entérique, où le noyau de pastille est formé de pastilles de départ de saccharose, de pantoprazole magnésium dihydraté ou de (-)-pantoprazole magnésium dihydraté, de carbonate de sodium, de PVP 25, d'amidon prégélatinisé et de dodécylsulfate de sodium, la couche intermédiaire est formée de HPMC, PVP 25, dioxyde de titane et d'oxyde de fer jaune, et l'enrobage entérique est formé d'Eudragit L 30 D et de citrate de triéthyle.

20. Forme pharmaceutique selon la revendication 19, dans laquelle les pastilles de départ de saccharose sont enduites avec une couche de pantoprazole magnésium dihydraté ou (-)-pantoprazole magnésium dihydraté, de carbonate de sodium, de PVP 25, d'amidon prégélatinisé et de dodécylsulfate de sodium.

21. Forme pharmaceutique selon la revendication 11, contenant entre 5 et 100 mg, du sel de magnésium de pantoprazole.

22. Forme pharmaceutique selon la revendication 21, qui contient une quantité du sel de magnésium de pantoprazole, qui correspond à 10, 20, 40, 50, 80 ou 100 mg de pantoprazole (acide libre).

23. Forme pharmaceutique selon la revendication 21, qui contient une quantité du sel de magnésium de pantoprazole, qui correspond à 40 mg de pantoprazole (acide libre).

24. Procédé de fabrication d'une pastille selon la revendication 1 par pulvérisation d'une suspension du délitant contenant la substance active et facultativement d'autres excipients sur des pastilles de départ et séchage des pastilles, de telle manière que le délitant soit de l'amidon de maïs prégélatinisé.

25. Procédé selon la revendication 24, dans lequel l'amidon prégélatinisé est de l'amidon de maïs prégélatinisé.

26. Procédé selon l'une quelconque des revendications 24 et 25, dans lequel la suspension est une suspension aqueuse du délitant et de la substance active.

27. Procédé pour fabriquer une forme pharmaceutique selon la revendication 11 par pulvérisation d'une suspension aqueuse d'amidon prégélatinisé contenant en outre du sel de magnésium de pantoprazole, du carbonate de sodium, du dodécylsulfate de sodium et du PVP en tant que liant sur les pastilles de départ, séchage des pastilles, enduction de celles-ci avec un sous-enrobage et un enrobage entérique, mélange avec des agents glissants le cas échéant et remplissage dans des capsules.

28. Procédé selon la revendication 27, dans lequel l'enrobage entérique est ajouté après séchage répété après couchage avec le sous-enrobage.

29. Procédé selon la revendication 28, qui est conduit dans un appareil à lit fluidisé.

30. Procédé pour fabriquer une forme pharmaceutique selon la revendication 11 par pulvérisation d'une suspension aqueuse d'amidon prégélatinisé contenant en outre du sel de magnésium de pantoprazole, du carbonate de sodium, du dodécylsulfate de sodium et de PVP en tant que liant sur des pastilles de départ, séchage des pastilles, enduction de celles-ci avec un sous-enrobage et un enrobage entérique, mélange avec des pastilles placebo et des agents glissants le cas échéant et remplissage dans des sachets de feuille d'aluminium.

31. Forme pharmaceutique selon la revendication 20, où la couche a une épaisseur comprise entre 80 et 140 µm, 90 et 135 µm, 95 et 130 µm, 100 et 125 µm.
